# EUROPEAN PATENT APPLICATION

(11) **EP 4 260 816 A1**
(43) Date of publication of application: **18.10.2023**
(21) Application number: 22315083.0
(22) Date of filing: 11.04.2022
(51) Int. Cl.: A61B 17/11

(54) **IMPLANT FOR MAGNETIC COMPRESSION ANASTOMOSIS**

(71) Applicant: BariaTek Medical, 75013 Paris (FR)
(72) Inventor: FRIEDRICH, Thomas, 01187 Dresden (DE); HEINTZE, Mario, 01309 Dresden (DE); ROKOSCH, Tobias, 01099 Dresden (DE); BIADILLAH, Joussef, 78600 Maisons-Laffitte (FR); GRAY, Jonathan, 75011 Paris (FR); NAZ, Christophe, 77300 Fontainebleau (FR)
(74) Representative: Hepp Wenger Ryffel AG

(57) **Abstract**

An implant for use in creating a magnetic compression anastomosis in a patient, the implant having a first low-profile configuration for insertion within a patient, and being deployable to a second operative configuration for use in creating an anastomosis, the implant comprising at least one supple polymer body (20, 20') and a plurality of magnetic elements (30) embedded at least partly within the polymer body, the polymer body being pliable to permit the polymer body to transition between a first state in the first configuration of the implant, and a second state in the second configuration of the implant. Plural polymer bodies may be coupled by a connecting element (24).

## Description

The present invention relates to an implant, a method of producing an implant and a method of delivering an implant for use in creating an anastomosis within a patient, especially in the digestive system, optionally joining two sections along the gastro-intestinal tract.

An anastomosis is considered to be the connection or opening between two normally separate structures and may be a natural occurrence in the human body, for example in the circulatory system, or may be surgically created by a trained practitioner. This may be the case, for example, in gastrointestinal procedures to promote weight loss in obese patients, such as Roux-en-Y procedures.

Although an anastomosis can be formed surgically by suturing or stapling, technical challenges remain in performing the same techniques laparoscopically or endoscopically, because access is considerably more restricted.

Implants and devices have been proposed to help avoid such complications. Magnetic compression anastomosis formation is one technique involving the use of magnets introduced on either side of neighbouring body lumen walls. Magnetic attraction compresses the tissue walls together, both joining the tissues and creating a mutual communication aperture at the join.

In EP 2 849 655 an implantable device is proposed for performing magnetic compression anastomosis and provides an exoskeleton and magnetic segments. The exoskeleton is made of a memory shape alloy and is designed to direct self-assembly of magnetic segments. WO 2016/183039 also describes different examples of self-opening magnetic rings, that spontaneously open to form a ring shape when delivered. One example includes spherical ball magnets within an extruded or moulded tube.

It would be desirable to improve on existing techniques for the creating an anastomosis, in particular, by magnetic compression.

### Summary of the invention:

A first aspect of the invention provides an implant for use in creating a magnetic compression anastomosis within a patient. The implant has a first low-profile configuration for insertion within a patient, and a second operative configuration for use in creating an anastomosis. The implant comprises a plurality of magnetic elements.

As used herein, the term "magnetic" refers to an element that behaves like a magnet or an element that can be attracted by a magnet. In some embodiments the magnetic elements may be any suitable elements that may induce a magnetic field, for example magnets. Additionally or alternatively, the magnetic elements may be any suitable elements that may be magnetically attracted, for example, they may be ferromagnetic elements.

The implant may further comprise any one, or any combination of any of, the following features, which are all optional:
(a) The implant may comprise at least one polymer body within which the plurality of magnetic elements is at least partly embedded. The polymer body may be at least partly pliable and/or bendable and/or flexible to permit the polymer body to transition between a first state in the first configuration of the implant and a second state in the second configuration of the implant.

In some embodiments, at least in the first state, the polymer body has a generally straight shape. Additionally or alternatively, in some embodiments, at least in the second state, the polymer body has an at least partly loop shape.

As used herein, the term "loop shape" means a shape that generally circumscribes a central area, whether or not the shape is round, or curved, or curvilinear, or a polygon comprising linear sides or segments. An at least partly loop shape is a shape that at least partly circumscribes a central area, even if the shape is not completely a closed shape, or is closed by non-polymeric material.

At least partly embedding the magnetic elements may help reduce risk of unwanted detachment of the magnetic elements from the implant. Magnetic elements can be difficult to attach securely in a biocompatible manner, especially to non-polymeric materials such as shape memory alloys. Loose magnetic elements are undesirable because they could cause internal injury and, in any event, they reduce the magnetic compression forces desired to be concentrated at the target side for anastomosis creation.

(b) The polymer body, if provided, may be made of any suitable polymer that may provide an at least partly pliable body. For example, the polymer body may be made of silicone.

Providing such a polymer body may allow for a soft, atraumatic implant and therefore may help avoid discomfort for the patient. It may also reduce the risk of damaging surrounding tissue. Additionally, using a polymer body may increase the pliability and/or the flexibility and/or the bendability of the implant. This may facilitate change in configuration of the implant to suit the anatomy at which the implant is placed, and/or to permit easier passage of the implant out of the body via the intestinal route, after the anastomosis has been created.

A polymer body may also be resistant to internal parameters of the gastrointestinal tract. For example, a polymer body may be more resistant to the acidic environment within the stomach than would be, for example, a shape memory alloy.

Using a polymer body may also permit economical production of the implant. Compared to shape memory alloy, for example, the material costs of a polymer may be relatively inexpensive. Furthermore, a polymer body may be easier to produce and consequently may allow for quicker production of the implants.

(c) The implant may comprise at least two (e.g. first and second) bodies within which magnetic elements are at least partly mounted or embedded, and wherein the bodies are interconnected, optionally by a connecting element that is integral with and/or integrally attached to both the first and second bodies. The connecting element may extend from an end of at least one body, optionally between ends of both first and second bodies.

At least one body may be a polymer body, optionally both first and second bodies are polymer bodies. Additionally or alternatively, at least one body may be pliable, optionally both first and second bodies are be pliable.

(d) The connecting element may be flexible. Optionally, and whether or not the connecting element is flexible, the connecting element may be reinforced by magnetic elements within the bodies (e.g. polymer bodies), and arranged at opposite ends of the connecting element. The magnetic elements may be chosen so that a repulsive magnetic force is applied between the connected portions of the bodies. A repulsive force may provide, or may enhance, column strength in the longitudinal direction of the connecting element, enabling a pushing force to be transmitted from one body to another.

(e) At least one polymer body may be moulded in a generally straight shape and/or may be resiliently deformable from and/or resiliently biased to a generally straight shape. Additionally or alternatively, at least one polymer body may be moulded in an at least partly loop-shape and/or may be resiliently deformable from and/or resiliently biased to an at least partly loop-shape.

Where at least a first polymer body has an at least partly loop shape, the body may self-bias into the loop shape when deployed in the body, and this may also be used to guide a second body into a matching loop shape by magnetic coupling between the bodies. For example, the second body may have a generally straight configuration, and be bendable or conformable into a loop shape, due at least in part to magnetic coupling with the first body.

Where at least one polymer body has a generally straight shape, this may assist in controllability when that body is deployed from a constraining sheath, because a straight shape may have less of a tendency itself to spring out from constraint by the sheath.

(f) The polymer body may be generally solid apart from regions occupied by the magnetic elements. Alternatively, the polymer body may have open space, e.g. a cavity, that is not occupied by a magnetic element. For example, the polymer body could be tubular.

(g) The polymer body may have first and second ends which are spaced apart from each other in the first state of the of the polymer body, and adjacent each other in the second state of the polymer body.

(h) The implant may comprise a positioning element or system to facilitate the positioning of at least one polymer body. For example, the positioning element may comprise a filament or tether attached to or integrated within the implant (e.g. attached to or integrated within a polymer body of the implant).

The positioning element or system may be self-adjusting, such it can function independently of manual input or control by an operator. For example, the positioning element may be elastic such that it applies an elastic bias to influence positioning during and/or after deployment. The elastic bias may, for example, increase during deployment.

Optionally, the positioning element or system may serve to maintain the position of a first polymer body whilst a second polymer body is positioned. For example, a filament or tether may extend from a position on the polymer body, the position being: spaced between opposite ends of the body; and/or spaced from a connecting element (if provided).

Additionally or alternatively, the positioning system element or system may serve to guide the polymer both to transition from the first state to the second state during deployment. For example, a filament may extend from one end of the polymer body towards an opposite end, so that tension in the filament draws one end towards another, to form an at least partial loop shape. The filament may, for example, pass through an aperture in the connecting element (if provided).

(i) At least one, preferably a majority, optionally substantially all, of the magnetic elements may be non-spherical magnetic elements. Especially in the case of the elements being magnets, use of non-spherical elements can provide significant advantages in being able to positively align the element in a certain magnetic orientation with respect to the polymer body, and thus with respect to other magnetic elements in the same body and/or within the implant. A non-spherical shape can have one or more lobes and/or sides and/or edges and/or corners that can form a keyed or fixed-position engagement with the adjacent or surrounding polymeric material. As well as assisting with assembly, such engagement can also maintain the magnetic alignment of the element in use, and resist any tendency for the alignment of the element to creep in use, compared to a spherical magnetic element which may have a tendency to roll or turn within its seat, towards a lowest-energy magnetic orientation.

In some embodiments, the magnetic elements may have a form selected as one or more from: generally cylindrical; generally oblong; generally elongate; generally polygonal; generally trapezoid, generally disc-shaped; generally flat; a form having at least one generally flat face, optionally multiple flat faces; a form having an exterior defined substantially entirely by flat faces.

(j) The magnetic elements may be at least partly spaced apart laterally from one another along the polymer body, at least in one state of the polymer body, e.g. in the first state and/or the second state. The magnetic elements may be spaced apart by intervening polymeric material of the polymer body.

(k) At least some of the magnetic elements may be arranged to avoid mutual attraction with laterally neighbouring magnetic elements at least in the first configuration, optionally in both the first and second configurations.

In some embodiments, an exception may exist at the opposite ends of the same polymer body (if provided) where magnetic elements may be configured to attract the ends into mutual end-to-end engagement to form an at least partial loop shape.

(l) Additionally or alternatively to any of the above, at least some, optionally a majority, optionally substantially all, of the magnetic elements may be arranged so that magnetic poles are aligned generally perpendicularly with respect to a longitudinal and/or loop direction of the implant and/or the polymer body (if provided).

(m) Additionally or alternatively, in some embodiments, the magnetic elements are arranged with alternating alignments of poles with respect to the longitudinal and/or loop direction of the implant and/or the polymer body (if provided). For example, neighbouring magnetic elements may be arranged with reverse magnetic orientations.

(n) The implant may be configured in such a manner that the compression of the internal tissue by the implant may induce necrosis of the tissue at the target site thus creating an anastomosis of the internal tissue.

Creating an anastomosis by magnetic compression may allow for reduced risks of complications that usually occur following an anastomosis. For example, the magnetic compression of tissues may facilitate the fusion of one or more tissue following necrosis of the tissues and thus may reduce the risk of anastomotic leakage.

A first low-profile configuration for insertion within the patient may facilitate the delivery of the implant within the patient. A low-profile may be smaller than the operational configuration and may be inserted using smaller instruments, and therefore, may help reduce the invasiveness of the procedure.

Additionally or alternatively to any of the above, the polymer body may be at least partly elongate and/or at least partly supple.

Having an at least partly elongate polymer body may help facilitate insertion of the implant within the patient. It may facilitate the change in configuration of the implant so as to create an anastomosis.

Having an at least parlty supple polymer body may allow for the implant to comply with contractions, for example, in the gastrointestinal tract. This may help reduce discomfort of the patient. A softer implant may further help to ease the evacuation of the implant once the anastomosis is complete.

In some embodiments, substantially all faces of the magnetic elements may be covered, at least partly, by the polymer body.

Covering the magnetic elements may avoid direct contact between internal tissue and the magnetic elements by providing a softer layer between internal tissue and the magnetic elements. This may also help reduce discomfort or irritation within the patient. This may further secure the magnetic elements with respect to the polymer body, and may avoid the detaching of the magnetic elements from the polymer body. This may prevent injury due to loose magnets within the body of the patient.

Additionally or alternatively, at least one polymer body may comprise an external sheath.

The external sheath may reduce friction or irritation between the implant and the internal tissue.

The external sheath may further contribute to securing the magnetic elements within one or more cavities with respect to the polymer body and may therefore reduce the risk of injuring the patient.

In some embodiments the implant may comprise at least two polymer bodies for creating an anastomosis within a patient. The magnetic elements of the first polymer body may magnetically attract the magnetic elements of at least a second polymer body.

Providing at least two polymer bodies with a plurality of magnetic elements may allow for a stronger magnetic compression of the internal tissue. It may also promote an even distribution of compressive forces along the profile of the implant. These characteristics may allow for a better anastomosis of the tissue.

In some embodiments at least a first polymer body may be positioned within a first tissue and at least a second polymer body may be positioned within a second tissue for anastomosing a plurality of tissues. Optionally the first tissue and second tissue may be at least partly adjacent one another, for example, the first tissue may be the stomach and a second tissue may be the intestines.

In some embodiments, at least two polymer bodies may be configured to at least partly superpose one another due to magnetic attraction.

The superposing of at least two polymer bodies may facilitate the compression of internal tissue for creating an anastomosis within a patient. It may facilitate the even compression of the internal tissue, and therefore, may help create the anastomosis.

Additionally or alternatively, at least two polymer bodies are interconnected, optionally by a connecting element that is integral with and/or integrally attached to each polymer body.

The extremities of the at least two polymer bodies connected by a connective element may comprise one or more magnetic elements.

If provided, the magnetic elements situated at the connected extremities of at least two polymer bodies may magnetically repulse one another.

The magnetic repulsion between the two polymer bodies may help to strengthen the connective element joining the at least two polymer bodies. This may help avoid deformation of the implant.

In some embodiments the change in configuration of the implant from a first configuration to a second configuration may compress at least two portions of internal tissue for creating an anastomosis of the internal tissue.

Compressing at least two portions of internal tissue may induce necrosis of the two portions of tissue where the implant is positioned and therefore may create for anastomosis between the two tissues. This may, for example, help connect two previously unconnected portions of tissue or may help bypass a portion of a tissue.

Additionally or alternatively, the implant may be at least partly linear in a first configuration and at least partly loop-shaped in a second configuration.

A loop-shape is considered to be any at least partly curvilinear and/or bent at least partly closed shape. For example and in no way exclusively, the implant may have a circular or polygonal shape.

An at least partly linear configuration the implant may allow for easier insertion within a patient at least partly linear configuration for insertion may allow the use of smaller instruments for delivering the implant. This may help provide an easier and less invasive insertion of the implant within a patient.

In some embodiments the loop-shape of the implant may be at least partly closed by magnetic attraction of a plurality of magnetic elements at the extremities of a polymer body.

Additionally or alternatively the magnetic elements at the extremities of two polymer bodies may be arranged to resist attraction between each other for helping the correct assembly of the polymer bodies in an at least partly closed loop-shape configuration.

An at least partly loop-shaped second configuration may help provide an even distribution of the compressive magnetic forces. This may avoid uneven compression of the tissue and may allow for quicker anastomosis of the tissue(s).

Connecting a first extremity and second extremity of the polymer body may help secure the second configuration and may help prevent the polymer body from returning to a first configuration.

In some embodiments the implant, optionally at least one polymer body of the implant, may have one or more grooves. The one or more grooves may be at least partly circumferential with respect of the implant.

The grove(s) may increase the flexibility of the implant. This may facilitate the adjusting of the implant form a first configuration to a second configuration. This may reduce the risk of tearing or damaging the implant whilst changing the configuration.

The implant may further comprise at least one positioning system for adjusting the configuration of the at least one polymer body. Optionally, the positioning system may be configured to attach to one or more polymer bodies. Optionally the positioning system may at least partly circumscribe at least one portion of one or more polymer bodies.

The positioning system may be any suitable means for adjusting an implant from a first configuration to a second configuration.

Additionally or alternatively the positioning system may have a first at least partly stretched configuration and a second non-stretched configuration. The positioning system may be configured to change from a stretched configuration to a second relaxed configuration; optionally the change in configuration of the positioning system may induce the change in configuration of the implant.

In some embodiments the positioning system may be at least partly elastic. The positioning system may elastically lengthen from a non-stretched configuration to a stretched configuration.

Additionally or alternatively the positioning system may self-adjust from a stretched position to a relaxed position for changing the configuration of the implant.

Optionally the positioning system may be in a first configuration when the implant is in a low-profile configuration and in a second configuration when the implant is in an operational configuration.

In some embodiments the positioning system may be further configured to maintain the position of a first polymer body with respect to the tissue whilst a second polymer body adjusts to the operative configuration under magnetic attraction with the first polymer body.

Including a self-adjusting positioning system may help reduce the number of instruments needed to position the implant. It may further help maintain the position of a first polymer body while a second polymer body is delivered and may therefore help make the procedure easier for the practitioner.

In a second aspect the invention provides a method of production of an implant for performing an anastomosis within a patient, optionally for creating an implant according to any preceding claims.

The method may comprise a step of moulding at least one polymer body.

In some embodiments a polymer body may be moulded into, but is not limited to, an at least partly linear shape.

Moulding the implant into an at least partly linear shape may facilitate the loading of an implant within a delivery device.

Additionally or alternatively a polymer body may be moulded into an at least partly loop-shape.

Moulding the polymer body into an at least partly loop-shape may allow for the implant to revert to the loop-shape when delivered to the target site. This may allow the forgoing of a positioning system and may thus help reduce costs or may allow for a less complicated procedure...

The polymer body may, in some embodiments, be moulded in such a manner that one or more cavities are provided.

Additionally or alternatively, a step consisting in integrating one or more magnetic elements to the polymer body may be provided.

In some embodiments the magnetics elements may be placed in one or more cavities of a pre-moulded polymer body. The cavities may be created due to the shape of the mould.

This may allow the magnetic elements to be placed once the polymer body has been moulded.

In some embodiments the cavities may be produced by moulding the polymer body around one or more magnetic elements placed within the mould.

Moulding the polymer body around the magnetic elements may help secure the elements with respect to the implant and may further help avoid detachment of the magnetic elements.

Further additionally or alternatively, the method may comprise a step of adding an additional layer to the polymer body for securing the one or more magnetic elements.

In some embodiments the additional layer may be moulded onto and/or placed around the pre-moulded polymer body.

The additional layer may help secure the plurality of magnetic elements with respect to the polymer body. This may help avoid unwanted detachment of the magnetic elements and may improve the safety of the implant.

In a third aspect the invention provides a method of delivering an implant, optionally for delivering an implant as described above.

The method comprises a step consisting of inserting the implant within a patient, optionally inserting the implant in a first low-profile configuration.

he method may provide a step for deploying the implant within the patient.

In some embodiments a first polymer body may be placed on a first side of a tissue, optionally positioned by use of the positioning system.

Additionally, if provided, at least a second polymer body may be delivered to a second side of the tissue, or to a second tissue, optionally in close proximity to the location of the first polymer body.

Further additionally or alternatively, a step for adjusting the configuration of the implant may be included. Optionally the implant is adjusted to a second configuration for anastomosing a tissue, the implant being adjusted to an at least partly loop-shaped configuration.

In some embodiments at least one polymer body of the implant may self-adjust to a second configuration under magnetic attraction forces from a previously positioned first polymer body.

Providing an implant that may at least partly self-adjust to an operational configuration may allow for an easier procedure and may reduce the need for additional instruments for changing the configuration of the implant. This may also decrease the complexity of the procedure.

Although certain features and ideas have been highlighted above and in the appended claims, protection is claimed for any novel feature or idea described herein and/or illustrated in the drawings whether or not emphasis has been placed thereon.

### Brief description of drawings:

Fig. 1 is a schematic side view of a first embodiment of implant in a low-profile configuration for use in creating a magnetic anastomosis within a patient.
Fig. 2 is a schematic perspective view of the implant of Fig. 1 in a deployed configuration for use in creating a magnetic anastomosis within a patient.
Fig. 3 is a schematic exploded side view of a second embodiment of implant for use in creating a magnetic anastomosis within a patient.
Fig. 4 is a schematic side view of a third embodiment of implant in a low-profile configuration for use in creating a magnetic anastomosis within a patient
Fig. 5 is a schematic perspective view of the third embodiment of implant in a partially deployed configuration for use in creating a magnetic anastomosis within a patient.
Fig. 6 is a schematic perspective view of a fourth embodiment of implant in its moulded configuration.
Fig. 7 is a schematic perspective view illustrating first and second bodies in loop shapes for forming a magnetic compression anastomosis.
Fig. 8 is a perspective view of mould tools, in a spaced apart arrangement, suitable for moulding polymer bodies of an implant.
Fig. 9 is a plan view of a mould tool of Fig. 8.
Fig. 10 is a schematic side view of the mould tools of Fig. 8 in assembled relation.

### Detailed description of some embodiments:

In the illustrations, the same reference numerals are used to denote the same or equivalent features amongst different embodiments and examples. Unless described to the contrary, the description of a feature in one embodiment may also apply to the same or equivalent feature in another embodiment or example. Features may also be interchanged in embodiments as desired.

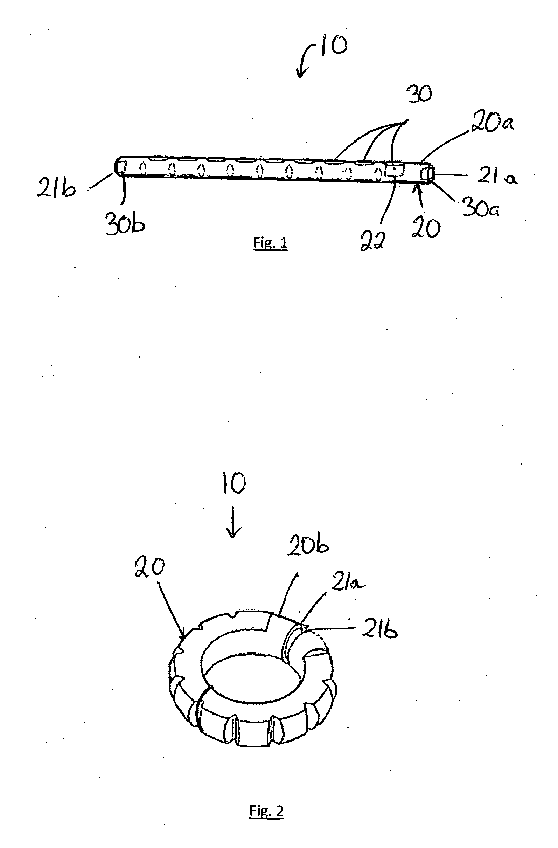

Referring to Figs. 1 and 2 a first embodiment of implant 10 configured for use in creating a magnetic compression anastomosis within a patient is shown. The implant 10 comprises a polymer body 20 having a plurality of cavities 22 configured to accommodate a plurality of magnetic elements 30. In Fig. 1, a mixture of magnetic elements 30 and open cavities 22 is shown schematically so that the arrangement of cavities can be seen.

The magnetic elements 30 may be magnets, or elements that behave like magnets or may be elements that can be attracted by magnets. The magnetic elements 30 may have any suitable shape. A non-spherical shape may be preferred to allow the magnetic element to lock in position, and/or to permit a predetermined magnetic orientation, and/or to prevent the orientation from changing in use, for example, due to rotation of the sphere in its seat.

The implant 10 has a first low-profile configuration 20a for insertion within a patient wherein the implant 10 is at least partly linear (see fig. 1). The implant 10 is further configured to be deployed into a second operative configuration 20b wherein the implant is at least partly loop-shaped (see fig.2).

The polymer body 20 of the implant has a first extremity (e.g. end) 21a and a second extremity (e.g. end) 21b. The polymer body 20 is at least partly flexible to facilitate changing from a first configuration 20a and a second configuration 20b.

In the second operative configuration 20b, a first extremity 21a of the polymer body 20 is positioned so as to be at least partly closer to a second extremity 21b of the polymer body 20.

The first extremity 21a and the second extremity 21b of the polymer body 20 are configured to connect to one another, optionally by magnetic attraction forces. Magnetic elements 30a and 30b may be arranged at the extremities 21a and 22b.

The implant 10 optionally further comprises a plurality of grooves 25. The grooves 25 are provided in an exterior circumferential surface to enhance the flexibility of the polymer body 20 to bend into the loop shape.

In use, the implant 10 is delivered into the body in the first configuration, and deployed into the second configuration, to form an at least partial loop shape, preferably a complete loop shape. The implant 10 is positioned on one side of target tissue in which an anastomosis is to be formed. A corresponding matching or complementary implant is delivered on other side of the tissue, and magnetic attraction between the implants compresses the tissue.

The polymer body may have a natural (e.g. moulded) shape, to which the polymer body is self-biased. The natural shape may be the first configuration, or the second configuration, or an intermediate shape between the first and second configurations.

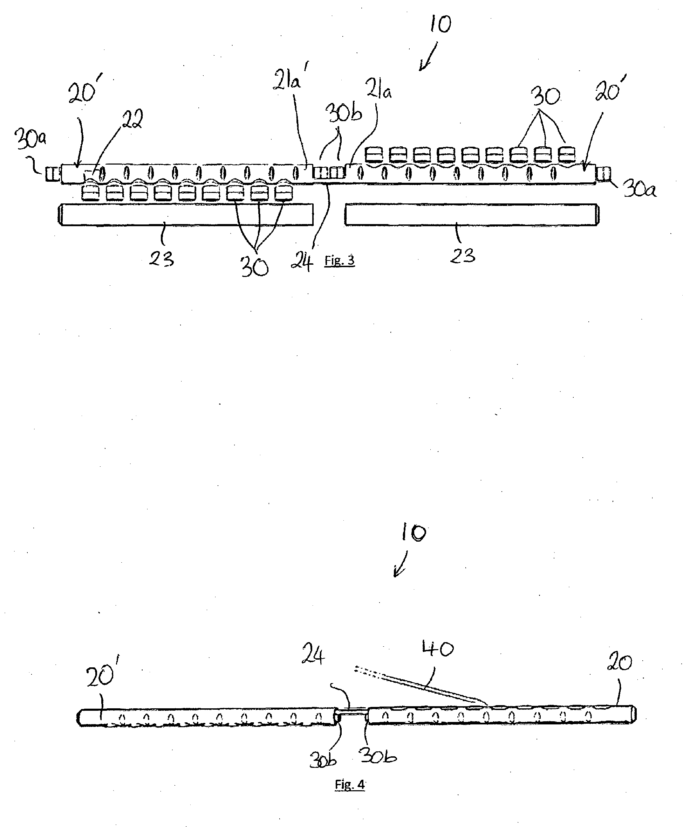

Referring to fig. 3, a second embodiment of implant comprises a first polymer body 20 and a second polymer body 20'. The implant is illustrated in a first low-profile configuration compatible for insertion within a patient. The two polymer bodies 20 and 20' are connected by a connecting element 24. In contrast to the first embodiment in which the implant provides only a single or individual loop shape, in the second embodiment, the implant provides both first and second loop shapes for fitting on opposite sides of tissue walls in which a magnetic compression anastomosis is to be formed.

The implant further comprises a plurality of magnetic elements 30 (see fig. 3). The magnetic elements 30 are configured to be at least partly embedded within a polymer body 20. In Fig. 3, the line within each magnetic element denotes schematically a division between magnetic poles. For example, for the majority of the magnetic elements 30, the magnetic poles are aligned generally perpendicularly with respect to a longitudinal axis and/or loop direction. Where the magnetic elements 30 are all magnets, in the first configuration, the magnetic orientation is the opposite of that desired for magnetic attraction. When the implant transitions to a second operative configuration, the plane of one polymer body may be folded over to overlie the other polymer body, turning one set of magnets upside-down, and into alignment for magnetic attraction.

Optionally the magnetic elements 30 are positioned in one or more cavities 22 of the implant 10. A cavity 22 may have at least one open end, and/or at least one closed end, and/or at least one end that is partly open. The closer the magnetic element 30 to the surface, the stronger the magnetic interaction. In some designs an open end may present the magnetic element generally flush with the surface of the polymer body. In the illustrated form, the cavities are generally open at one end only. For example, the cavities 22 of the first polymer body 20 open in one direction (e.g. downwardly, in the example shown), and the cavities 22 of the second polymer body 20' open in an opposite direction (e.g. upwardly, in the example shown). When the implant transitions to a second operative configuration, the plane of one polymer body may be folded over to overlie the other polymer body, bringing matching ends (e.g. open ends) of the cavities 22 facing towards one another.

The connecting element 24 is at least partly flexible. The connecting element 24 is configured to connect a first extremity 21a of a first polymer body 20 with a first extremity 21a'of a second polymer body 20'. The flexibility allows one polymer body to be folded over to overlie the other polymer body when the device transitions from its first, low-profile configuration for delivery, to its second, operative configuration for implantation.

The connecting element 24 is reinforced by a plurality of magnetic elements 30a and 30b placed at a first extremity 21a of a first polymer body 20 and at a first extremity 21a'of a second polymer body 20' (see fig.3). The magnetic elements at the first extremities 21a and 21a' are arranged to avoid attraction between one another, preferably to repel each other. This can increase the effective column strength of the implant across the flexible connecting element.

The implant as illustrated in fig.3 further comprises at least one sheath 23, optionally two sheaths. Optionally, the sheath 23 is configured to cover at least one polymer body 20; 20' of the implant 10. The sheath may, for example, be made of a heat-shrink polymer, e.g. tubular film, that can be heat-shrunk around the polymer body. The sheath may serve to further lock the magnetic elements captive within the polymer bodies. In the illustrated form, two sheaths are provided, one for each polymer body, in order not to impede foldability of the connecting element.

As seen in fig.4 the implant 10 has a positioning system 40, in the form of at least one filament or tether. The positioning system is connected to at least one polymer body 20; 20'. In use, the positioning system 40 may serve to assist positioning of one of the polymer bodies, for example, a distal-most polymer body, to lie against a tissue surface on an opposite side of the tissue walls from the remainder of a delivery system. The delivery system may comprise a resilient filament that biases the polymer body as the filament extends elastically with deployment of the implant.

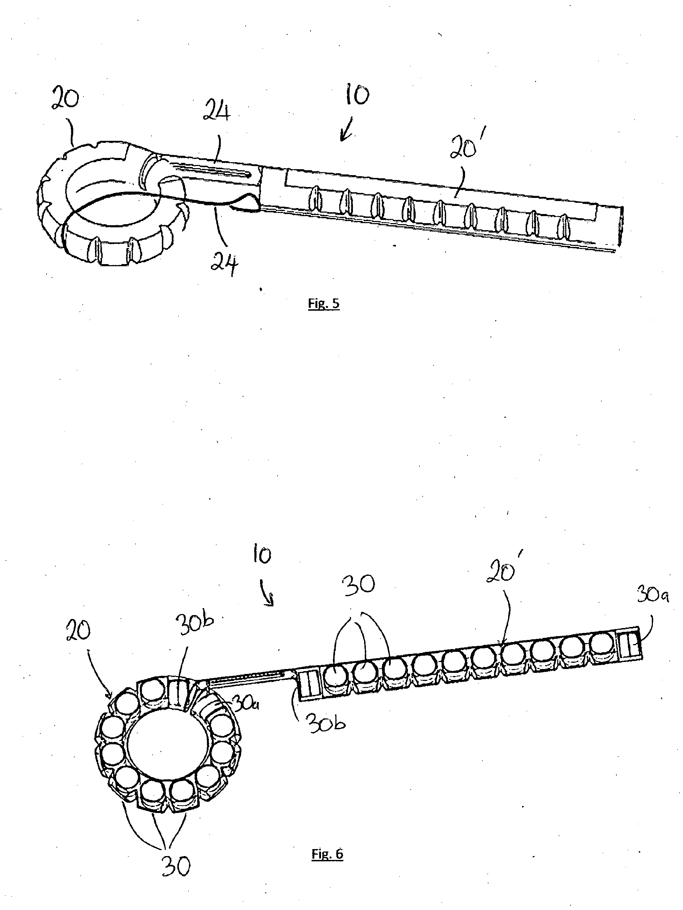

Figs. 5 and 6 depict an alternative embodiment of the implant comprising a first polymer body 20 and second polymer body 20'. They further show an alternative embodiment of a positioning system 40. The positioning system 40 is attached to the first (e.g. distal) polymer body 20.

The principal difference compared to the preceding embodiment is that the first and second polymer bodies are moulded in different shapes. The first polymer body 20 is moulded to have a natural curved or loop shape to which the polymer body will automatically be self-biased when deployed from a delivery catheter (e.g. on an opposite side of tissue to the remainder of the delivery catheter). This can assist in correct deployment of the first polymer body on the remote side of tissue. The second polymer body 20' is moulded to have a natural straight (or at least less-curved) shape. This may avoid assist fine control of deployment of the second polymer body from the delivery catheter, and reduce any tendency for the second polymer body 20' self-ejecting from the delivery catheter when the implant reaches near full deployment. The second polymer body 20' may adopt the same shape as the first polymer body 20 as it progressively deploys from the delivery catheter, due to magnetic attraction with the first body 20.

The implant of fig. 6 further shows a plurality of magnetic elements 30 embedded within the first polymer body 20 and within the second polymer body 20'. The first polymer body has been deployed to an operational configuration and the second polymer is in a low-profile configuration. The implant has magnetic elements embedded within the first polymer body 20 and second polymer body 20'. The magnetic elements in the embodiment shown by fig.6 are cylindrical.

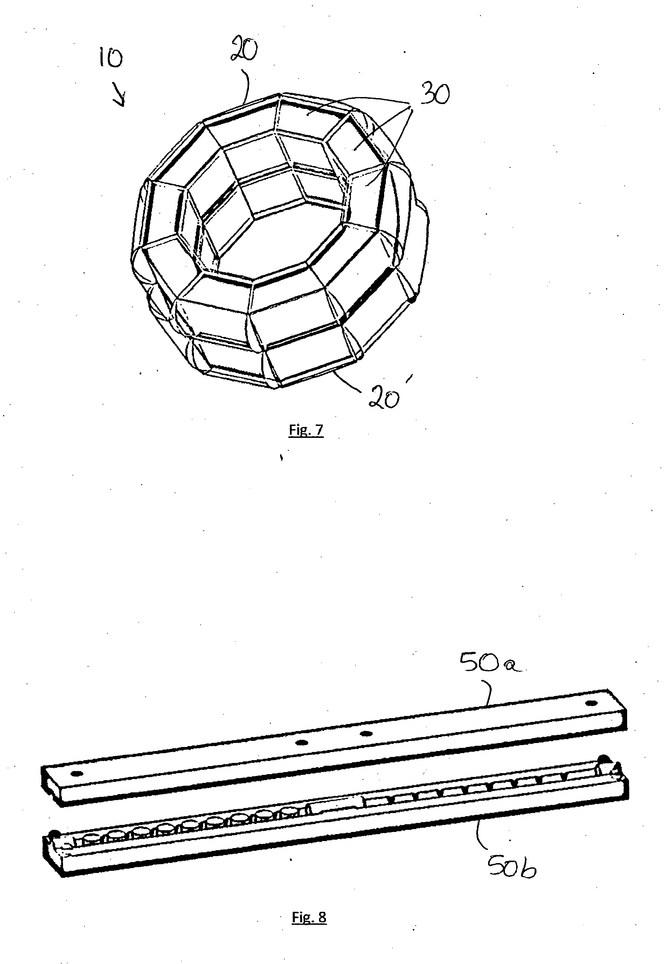

Illustrated in fig. 7 in some alternative embodiments the implant has a plurality of trapezoid magnetic elements embedded within respective polymer bodies 20, 20'. This embodiment may include any of the features described above.

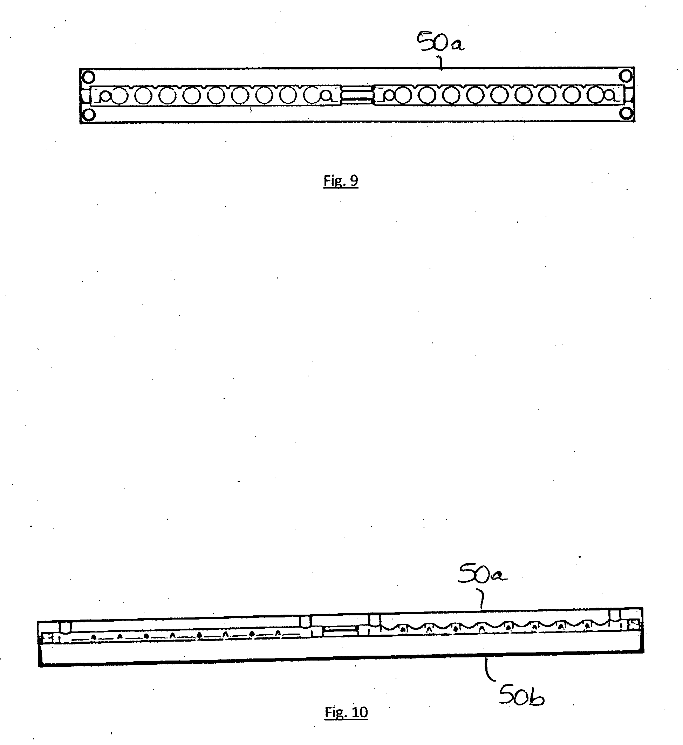

Figs. 8 to 10 illustrate mould tools 50a and 50b for moulding an polymer bodes 20 and 20' of an implant 10. In the illustrated form, the mould tools are shown in a generally straight elongate configuration.

It will be appreciated that the foregoing description is merely illustrative of examples of the invention, and does not limit the scope of protection.

## Claims

1. An implant for use in creating a magnetic compression anastomosis in a patient, the implant having a first low-profile configuration for insertion within a patient, and being deployable to a second operative configuration for use in creating an anastomosis, the implant comprising at least one polymer body and a plurality of magnetic elements embedded at least partly within the polymer body, the polymer body being pliable to permit the polymer body to transition between a first state in the first configuration of the implant, and a second state in the second configuration of the implant.

2. Implant according to any preceding claim, wherein at least one polymer body is at least partly elongate and at least partly supple.

3. Implant according to claim 2, wherein magnetic elements disposed at opposite ends of the polymer body are configured to attract to hold the polymer body in a loop shape by magnetic attraction.

4. Implant according to any preceding claim, wherein substantially all faces of the magnetic elements are covered, at least partly, by the polymer body.

5. Implant according to any preceding claim wherein the at least one polymer body further comprising an external sheath.

6. Implant according to any preceding claim, wherein at least one, optionally a plurality, optionally a majority, optionally substantially all, of the magnetic elements are non-spherical in shape.

7. Implant according to any preceding claims wherein the implant comprises at least two polymer bodies for creating an anastomosis within a patient, each polymer body having a plurality of magnetic elements such that magnetic elements of one polymer body generate an attraction force with respect for magnetic elements of another polymer body.

8. Implant according to claim 6 wherein the at least two polymer bodies are configured to superpose one another under magnetic attraction of the magnetic elements.

9. Implant according to claims 6 or 7 wherein the at least two polymer bodies are interconnected, optionally by a connecting element that is integral with and/or integrally attached to each polymer body.

10. Implant according to claim 9, wherein in the first configuration of the implant, a magnetic element in a first of the polymer bodies is configured to repel a magnetic element in a second of the polymer bodies, the magnetic elements being disposed at opposite ends of the connecting element.

11. Implant according to any of claims 6 to 8 wherein at least one polymer body is biased to its first configuration, and at least one polymer body is biased to its second configuration.

12. Implant according to any preceding claims, wherein the implant is at least partly linear in a first configuration and at least partly loop-shaped in a second configuration.

13. Implant according to claim 12, wherein at least one polymer body is biased its first configuration, and at least one polymer body is biased to its second configuration.

14. Implant according to any preceding claim, wherein the implant further comprises at least one positioning system for adjusting the configuration of the at least one polymer body, optionally wherein the positioning system comprises a resilient element for biasing the polymer body with extension of the resilient element.

15. Method of production of an implant for performing an anastomosis within a patient, optionally for creating an implant according to any preceding claims, wherein the method comprises one or more of, and in any order:
(i) moulding at least one polymer body;
(ii) integrating one or more magnetic elements to the at least one polymer body;
(iii) adding an additional layer to the polymer body for securing the one or more magnetic elements.
